# EUROPEAN PATENT SPECIFICATION

(11) **EP 2 477 599 B1**
(45) Date of publication and mention of the grant of the patent: **26.10.2016**
(21) Application number: 10755068.3
(22) Date of filing: 15.09.2010
(51) Int. Cl.: A61K 8/81, A61K 8/891, A61Q 5/02, A61Q 19/10

(54) **SILICONE DEPOSITION AID FOR PERSONAL CARE COMPOSITIONS**
SILIKONABSCHEIDUNGSHILFE FÜR KÖRPERPFLEGEZUSAMMENSETZUNGEN
AUXILIAIRE DE DÉPÔT DE SILICONE POUR COMPOSITIONS DE SOINS D'HYGIÈNE ET DE BEAUTÉ

(30) Priority: 15.09.2009 US 242505 P
(43) Date of publication of application: 25.07.2012
(73) Proprietor: Dow Global Technologies LLC, Midland, MI 48674 (US)
(72) Inventor: JORDAN, Susan, Doylestown PA 18902 (US); STRANDBURG, Gary, Mount Pleasant MI 48858 (US); CHURCHFIELD, Mechelle, Midland MI 48642 (US)
(74) Representative: Beck Greener
(86) International application number: PCT/US2010/048860
(87) International publication number: WO 2011/034877

(56) References cited:
- EP-A2- 1 481 661
- WO-A1-03/028678
- WO-A1-03/028680
- WO-A1-03/028682
- WO-A1-2009/047106
- WO-A1-2009/064739
- US-A- 5 409 695
- US-A1- 2003 044 373

## Description

### Field

The present application relates to personal care compositions.

### Background

Silicone deposition is known to increase perceptions of smoothness in personal care compositions, particularly conditioners, shampoos, and body washes. See, for example, US 2003/0044373, disclosing hair treatment compositions comprising a surfactant, a dispersed insoluble benefit agent, and PTFE microparticles and also describing the use of PTFE microparticles as a deposition aid for a dispersed insoluble benefit agent in an aqueous hair treatment composition. However, it is a major challenge in the art to balance the personal care components in a way that promotes silicone deposition, as opposed to encouraging the silicone to wash off. Thus, what are needed are deposition aids to increase silicone deposition. Thus, it is an important goal in the art to create new formulations.

### Summary

In one embodiment, the present invention provides personal care compositions, comprising an aqueous dispersion comprising silicone and an ethylene acrylic acid copolymer; and at least one cosmetically acceptable surfactant, emollient, or cosmetic active.

### Detailed Description

In one embodiment, the present invention provides personal care compositions, comprising an aqueous dispersion comprising silicone and an ethylene acrylic acid copolymer; and at least one cosmetically acceptable surfactant, emollient, or cosmetic active.

"Personal care" relates to compositions to be topically applied to a person (including mouth, ear, and nasal cavities, but not ingested). Examples of personal care compositions include skin care products (e.g., facial cream, moisturizers, leave on and rinse off lotions, sunscreens, foundation, mascara, eye-liner, lipstick, cleansers, and the like) and hair care products (including shampoos, leave on and rinse off conditioners, styling gels and hairsprays). Preferably, the personal care composition is a shampoo, a rinse-off conditioner, a leave-in conditioner, or a body wash.

"Cosmetically acceptable" refers to ingredients typically used in personal care compositions, and is intended to underscore that materials that are toxic when present in the amounts typically found in personal care compositions are not contemplated as part of the present invention.

Copolymerizing ethylene with acrylic acid yields ethylene-acrylic acid (EAA) copolymers, which are known as flexible thermoplastics for blister packaging and the like. A preferred ethylene acrylic acid copolymer comprises greater than about 15 wt% acrylic acid, preferably greater than about 17 wt% acrylic acid, more preferably about 20 wt% acrylic acid. It should be understood that ranges recited in this disclosure include all subcombinations of ranges.

A preferred EAA copolymer is PRIMACOR 5990 copolymer (20 wt% acrylic acid), which has a melt index of 1300 g/10 minute (ASTM Method D-1238 at 190° C) and a Brookfield viscosity of 13,000 cps at 350° F, and is available from The Dow Chemical Company. Another preferred EAA copolymer is PRIMACOR 5980i copolymer (20.5 wt% acrylic acid), which has a melt index of 300 g/10 minute (ASTM Method D-1238 at 190° C), available from The Dow Chemical Company. EAA copolymers are also available under the tradename NUCREL 2806, available from E.I. du Pont de Nemours and Company, Inc. Ethylene-acrylic acid and ethylene-methacrylic acid copolymers, are described in U.S. Pat. Nos. 4,599,392, 4,988,781, and 5,938,437.

Silicone includes silicone oils, for instance volatile or non-volatile polymethylsiloxanes (PDMS) comprising a linear or cyclic silicone chain, which are liquid or pasty at room temperature, especially cyclopolydimethylsiloxanes (cyclomethicones) such as cyclopentasiloxane and cyclohexadimethylsiloxane, polydimethylsiloxanes comprising alkyl, alkoxy or phenyl groups, which are pendent or at the end of a silicone chain, these groups comprising from 2 to 24 carbon atoms, phenyl silicones, for instance phenyl trimethicones, phenyl dimethicones, phenyltrimethylsiloxydiphenylsiloxanes, diphenyl dimethicones, diphenylmethyldiphenyltrisiloxanes 2-phenylethyltrimethyl siloxysilicates and polymethylphenylsiloxanes, fluoro oils such as partially hydrocarbon-based and/or partially silicone-based fluoro oils, preferably dimethicone. A preferred blend of dimethicone, Laureth-23, and C 12-15 Pareth-3 is commercially available from Dow-Corning under the tradename DOW CORNING 2-1491 Silicone Emulsion, also described as a 60% large particle size non-ionic emulsion of a blend of ultra-high molecular weight polydimethylsiloxane gum and intermediate molecular weight polydimethylsiloxane fluid.

In one embodiment, the ethylene acrylic acid copolymer and silicone are melt-kneaded in an extruder along with water and a neutralizing agent, such as ammonia, potassium hydroxide, or a combination of the two, to form an aqueous dispersion.

Mechanical dispersion, such as a Parr reactor, is used to create the aqueous dispersion.

The ethylene acrylic acid copolymer is present in a range from about 0.5 wt% to about 15 wt% by weight of the aqueous dispersion, preferably in a range from about 2 wt% to about 10 wt%.

In one embodiment, the silicone is present in a range from about 10 wt% to about 50 wt% by weight of the aqueous dispersion, for example in a range from 5 wt% to 35 wt% preferably in a range from 10 wt% to 25 wt%, or preferably in a range from about 20 wt% to about 30 wt%.

Preferably, the ethylene acrylic acid copolymer and silicone is in a polymer ratio of about 1:4, more broadly, in a range from about 1:2 to about 1:8. The solids content of the aqueous dispersion is in a range from about 10% by weight to about 30% by weight, preferably about 20% by weight.

In turn, the aqueous dispersion is present in a range from about 0.1 wt% to about 10 wt% of solids, preferably about 0.5 wt% to about 5 wt%, by weight of the personal care composition.

In one embodiment, the composition further comprises a cationic polymer. Cationic polymer is herein defined as a polysaccharide modified to have a positive charge, for example, cationic cellulose derivatives (including for example PQ 10, PQ 24, and PQ 67), cationic guar derivatives, cationic methacrylamido polymers, and synthetic cationic polymers, such as PQ6 and PQ7. In a preferred embodiment, the cationic polymer is cationically modified hydroxyethylcellulose, which is commercially available from The Dow Chemical Company under the tradename UCARE.

Alternatively, in one embodiment, the personal care composition is substantially free of cationic polymer, i.e., a polysaccharide modified to have a positive charge, for example, cationic cellulose derivatives or cationic guar derivatives

In one embodiment, the surfactant is an anionic, nonionic, or amphoteric surfactant, or a mixture thereof.

In one embodiment, the surfactant is a detergent surfactant. In this embodiment, the surfactant is present in an amount from 1 wt% to 30 wt% by weight of the composition, for example from about 2% to about 30% by weight of the composition, preferably from about 5% to about 25% by weight of the composition, most preferably from about 7% to about 20% by weight of the composition.

Preferably, the detergent surfactant is an anionic surfactant in combination with an amphoteric surfactant. In one embodiment, the anionic surfactant is ammonium laureth sulfate, ammonium lauryl sulfate, sodium laureth sulfate, or sodium lauryl sulfate.

In one embodiment, the mixture is an anionic surfactant in combination with a second surfactant that is disodium cocoamphodiacetate, decylglucoside, or cocamidopropyl betaine.

In a preferred embodiment, the surfactant is a mixture of sodium laureth sulfate (such as is commercially available from Cognis as under the tradename STANDAPOL ES) and disodium cocoamphodiacetate (such as is commercially available from Henkel as under the tradename VELVETEX CDC). When the surfactant is a mixture of sodium laureth sulfate and disodium cocoamphodiacetate, the ratio of sodium laureth sulfate to disodium cocoamphodiacetate is in a range from about 9:1 to about 2:1, most preferably about 6:1.

In one embodiment, the composition includes citric acid to adjust the pH.

Other optional ingredients for personal care compositions of the present invention include cosmetically acceptable emollients, sunscreens, surfactants, emulsifiers, preservatives, rheology modifiers, colorants, dyes, preservatives, pH adjustors, propellants, reducing agents, fragrances, foaming agents, tanning agents, depilatory agents, flavors, astringents, antiseptics, deodorants, antiperspirants, insect repellants, bleaches, lighteners, anti-dandruff agents, adhesives, polishes, strengtheners, fillers, barrier materials, or biocides.

In some embodiments, the personal care composition further comprises an optional rheology modifier as a thickener. Examples of thickeners include polymers, for example, modified or unmodified carboxyvinyl polymers, such as the products sold under the names CARBOPOL and PEMULEN (INCI name: Acrylates/C₁₀₋₃₀ alkyl acrylate crosspolymer; available from Noveon), polyacrylates and polymethacrylates, such as the products sold under the names LUBRAJEL and NORGEL (commercially available from Guardian) or HISPAGEL (commercially available from Hispano Chimica), polyacrylamides, 2-acrylamido-2-methylpropanesulfonic acid polymers and polymers, which are optionally crosslinked and/or neutralized, for instance the poly(2-acrylamido-2-methylpropane-sulfonic acid) sold by Clariant (INCI name: ammonium polyacryldimethyltauramide), emulsified crosslinked anionic polymers of acrylamide and AMPS, such as those sold under the name SEPIGEL 305 (INCI name: Polyacrylamide/C13-14 Isoparaffin/Laureth-7; from Seppic) and under the name SIMULGEL 600 (INCI name: Acrylamide/Sodium acryloyldimethyltaurate polymer/Isohexadecane/Polysorbate 80; from Seppic), polysaccharide biopolymers, for instance xanthan gum, guar gum, carob gum, acacia gum, scleroglucans, chitin and chitosan derivatives, carrageenans, gellans, alginates, celluloses such as microcrystalline cellulose, cellulose derivatives, associative polymers, for instance associative polyurethanes, polymers comprising at least two hydrocarbon-based lipophilic chains comprising from 6 to 30 carbon atoms, separated with a hydrophilic sequence, such as the polyurethanes sold under the names SERAD FX1010, SERAD FX1100 and SERAD FX1035 (commercially available from Hüls America), RHEOLATE 255, RHEOLATE 278 and RHEOLATE 244 (INCI name: Polyether-urea-polyurethane; from Rheox), DW 1206F, DW 1206J, DW 1206B, DW 1206G, and ACRYSOL RM 2020 (commercially available from Röhm & Haas).

Colorants include pigments, which are used especially in make-up, including metal oxide pigments, titanium dioxide, optionally surface-treated, zirconium oxide or cerium oxide, zinc oxide, iron oxide (black, yellow or red), chromium oxide, manganese violet, ultramarine blue, chromium hydrate and ferric blue, carbon black, pigments of barium, strontium, calcium or aluminum (for example D&C or FD&C), cochineal carmine, mica coated with titanium or with bismuth oxychloride, titanium mica with iron oxides, titanium mica with, especially, ferric blue or chromium oxide, titanium mica with an organic pigment, nacreous pigments based on bismuth oxychloride, goniochromatic pigments, for example pigments with a multilayer interference structure, reflective pigments, for example particles with a silver-coated glass substrate, glass substrate coated with nickel/chromium/molybdenum alloy, glass substrate coated with brown iron oxide, particles comprising a stack of at least two polymer layers, for instance MIRROR GLITTER (commercially available from 3M).

Dyes include water-soluble dyes such as copper sulfate, iron sulfate, water-soluble sulfopolyesters, rhodamines, natural dyes, for instance carotene and beetroot juice, methylene blue, caramel, the disodium salt of tartrazine and the disodium salt of fuschin, and mixtures thereof. Liposoluble dyes from the list above may also optionally be used.

Preservatives include alcohols, aldehydes, methylchloroisothiazolinone and methylisothiazolinone, p-hydroxybenzoates, and in particular methylparaben, propylparaben, glutaraldehyde and ethyl alcohol.

The pH adjustors, include inorganic and organic acids and bases and in particular aqueous ammonia, citric acid, phosphoric acid, acetic acid, and sodium hydroxide.

Reducing agents include ammonium thioglycolate, hydroquinone and sodium thioglycolate.

Fragrances may be aldehydes, ketones, or oils obtained by extraction of natural substances or synthetically produced as described above. Often, fragrances are accompanied by auxiliary materials, such as fixatives, extenders, stabilizers and solvents.

Biocides include antimicrobials, bactericides, fungicides, algaecides, mildicides, disinfectants, antiseptics, and insecticides.

The amount of optional ingredients effective for achieving the desired property provided by such ingredients can be readily determined by one skilled in the art.

In a preferred embodiment, the personal care composition is a shampoo, body wash, or facial cleanser, preferably a shampoo.

The amount of optional ingredients effective for achieving the desired property provided by such ingredients can be readily determined by one skilled in the art.

In use, the personal care compositions are applied to hair or skin.

### Examples

The following examples are for illustrative purposes only and are not intended to limit the scope of the present invention. All percentages are by weight unless otherwise specified.

### Example 1

Personal care compositions of the present invention include aqueous dispersions comprising an ethylene acrylic acid copolymer. Examples of such aqueous dispersions include the following:
Batch 1: PRIMCAOR™5980i resin (30.1g), potassium hydroxide (3.9g), and water (90g) are placed in a Parr reactor vessel fitted with a Cowles blade. The material is heated to about 120°C while mixing slowly. Once the desired temperature is reached, the mixer is run on high (∼1800 rpm) for about 25 minutes. The mixture is allowed to cool with stirring until reaching at least 30°C.

46.5g of this mixture is combined with Dow Corning DC-200 polydimethylsiloxane (∼60,000 cSt) (42g) and placed in a Parr reactor, mixing on high (∼1800 rpm) at room temperature for about 25 minutes. While still mixing on high, the sample is diluted to a desired solids concentration of about 20% solids with water fed into the reactor with an HPLC pump. The final aqueous dispersion is then collected.

### Example 2

Exemplary personal care shampoo compositions contain the components recited in TABLE 1 on a weight/weight basis (wt. %).

**TABLE 1**

| | **Batch A** | **Batch B** | **Batch C (reference example)** |
|---|---|---|---|
| STANDAPOL ES-2 sodium laureth sulfate | 60.78 | 60.78 | 60.78 |
| VEL VETEX CDC disodium cocoamphodiacetate | 6.92 | 6.92 | 6.92 |
| LEXEMUL EGDS pearlizing agent | 2.0 | 2.0 | 2.0 |
| JAGUAR C-13S cationic guar (2%) | -- | -- | 12.5 |
| Batch 1 | 5.0 | 2.5 | 5.0 |
| Citric Acid (10%) | 2.1 | 2.1 | 2.1 |
| GLYDANT DMDM Hydantoin | 0.4 | 0.4 | 0.4 |
| Deionized water | q.s. | q.s. | q.s. |

Combine Batch 1 with base surfactants. Slowly heat to 74°C while mixing with an overhead stirrer (approximately 500 rpm). Continue stirring until surfactants are in solution. Add pearlizing agent at approximately 500rpm, maintaining speed for 15 minutes, then cool slowly to about 35°C. Increase stirrer speed to 750 ppm and add Batch 1. Stir for about 15 minutes. Slowly add cationic polymer and stir about 30 minutes. Add 10% citric acid and stir about 10 minutes. Add Glydant preservative and q.s. with water to 100 g. Stir 15 minutes at approximately 500 rpm.

### Example 3 (Comparative)

A conventional shampoo composition contains the components recited in TABLE 2 on a weight/weight basis (wt. %).

**TABLE 2**

| | **Comparative Batch Z** |
|---|---|
| STANDAPOL ES-2 sodium laureth sulfate | 60.78 |
| VELVETEX CDC disodium cocoamphodiacetate | 6.92 |
| LEXEMUL EGDS pearlizing agent | 2.0 |
| JAGUAR C-13S cationic guar (2%) | 12.5 |
| DC 1664 trimethyl-trimethylsilyloxy-silane (50%) | 2.0 |
| Citric Acid (10%) | 2.1 |
| GLYDANT DMDM Hydantoin | 0.4 |
| Deionized water | q.s. |

Combine silicone with base surfactants. Slowly heat to 74°C while mixing with an overhead stirrer (approximately 500 rpm). Continue stirring until surfactants are in solution. Add pearlizing agent at approximately 500rpm, maintaining speed for 15 minutes, then cool slowly to about 35°C. Increase stirrer speed to 750 ppm and add Batch 1. Stir for about 15 minutes. Slowly add cationic polymer and stir about 30 minutes. Add 10% citric acid and stir about 10 minutes. Add Glydant preservative and q.s. with water to 100 g. Stir 15 minutes at approximately 500 rpm.

### Example 4

Compositions substantially according to the protocols of Examples 2 and 3 were prepared. Pre-washed and pre-hydrated tresses of European virgin brown or 8-hour bleached hair (available from International Hair Importers and Products Inc.) were treated with 0.5 g of these shampoo formulations. The shampoo was worked into the hair for 1 min. and then rinsed off under running tap water at 38°C at 0.4 gal./min. water flow.

The hair tresses were hung and dried. After the tresses were completely dried, dry sensory evaluations were conducted. Ten expert panelists trained to evaluate the performance of cosmetic products on hair were asked to evaluate comb-ability and feel in the dry stage. Each panelist evaluated a pair of tresses, one tress treated with a composition of the invention versus one treated with a comparative composition. The panelists were asked to pick one tress with superior attributes. Results are given in **TABLE 3.**

**TABLE 3**

| | | **Batch B vs. Comp. Batch Z** |
|---|---|---|
| Brown Hair | Dry comb | 40 |
| | Dry feel | 40 |
| Bleached Hair | Dry comb | 60 |
| | Dry feel | 60 |

The results show that even at relatively low concentration, the inventive formulations provide statistically the same or better conditioning as silicone polymers.

Silicone deposition was determined for Batch A, Batch C (reference example), and Comparative Batch Z. Batch A had 1066 ±58 ppm silicone deposited, Comparative Batch Z had 872 ±46 ppm silicone deposited, and Batch C had 261 ±4 ppm silicone deposited. Moreover, wet and dry panel studies indicated a clear preference for Batch A over Batch C on both virgin and bleached hair. Accordingly, in the present invention, the personal care composition is substantially free of cationic polymer (for example, cationic guar).

It is understood that the present invention is not limited to the embodiments specifically disclosed and exemplified herein. Various modifications of the invention will be apparent to those skilled in the art. Such changes and modifications may be made without departing from the scope of the appended claims.

Moreover, each recited range includes all combinations and subcombinations of ranges, as well as specific numerals contained therein.

## Claims

1. Use of an aqueous dispersion comprising silicone and an ethylene acrylic acid copolymer for increasing the deposition of silicone on hair from a personal care composition comprising said aqueous dispersion and at least one cosmetically acceptable surfactant; wherein the personal care composition has no cationic polymer.

2. The use of claim 1, wherein the silicone is present in a range from 5 wt% to 35 wt% by weight of the aqueous dispersion, preferably in a range from 10 wt% to 25 wt%.

3. The use of claim 1, wherein the ethylene acrylic acid copolymer is present in a range from 0.5 wt% to 15 wt% by weight of the aqueous dispersion, preferably in a range from 2 wt% to 10 wt%.

4. The use of claim 1, wherein the aqueous dispersion is present in a range from 0.1 wt% to 10 wt% of solids by weight of the personal care composition, preferably in a range from 0.5 wt% to 5 wt%.

5. The use of claim 1, wherein the surfactant is present in a range from 1 wt% to 30 wt% by weight of the personal care composition, preferably from 5 wt% to 25 wt%, and more preferably from 7 wt% to 20 wt%.

6. The use of claim 1, wherein the personal care composition is a shampoo or a body wash composition.

7. A method of increasing the deposition of silicone on hair, comprising:
dispersing the silicone in an aqueous dispersion comprising an ethylene acrylic acid copolymer;
including the aqueous dispersion in a personal care composition that has no cationic polymer; and
applying the personal care composition to hair.

8. The method of claim 7, wherein the deposition is increased by at least 20%.

## Patentansprüche

1. Die Verwendung einer wässrigen Dispersion, die Silikon und ein Ethylen-Acrylsäurecopolymer zum Erhöhen der Ablagerung von Silikon auf Haar von einer Körperpflegezusammensetzung beinhaltet, die die wässrige Dispersion und mindestens ein kosmetisch akzeptables Tensid beinhaltet;
wobei die Körperpflegezusammensetzung kein kationisches Polymer aufweist.

2. Verwendung gemäß Anspruch 1, wobei das Silikon in einem Bereich von 5 Gew.-% bis 35 Gew.-% der wässrigen Dispersion, vorzugsweise in einem Bereich von 10 Gew.-% bis 25 Gew.-% vorhanden ist.

3. Verwendung gemäß Anspruch 1, wobei das Ethylen-Acrylsäurecopolymer in einem Bereich von 0,5 Gew.-% bis 15 Gew.-% der wässrigen Dispersion, vorzugsweise in einem Bereich von 2 Gew.-% bis 10 Gew.-% vorhanden ist.

4. Verwendung gemäß Anspruch 1, wobei die wässrige Dispersion in einem Bereich von 0,1 Gew.-% bis 10 Gew.-% an Feststoffen pro Gewicht der Körperpflegezusammensetzung, vorzugsweise in einem Bereich von 0,5 Gew.-% bis 5 Gew.-% vorhanden ist.

5. Verwendung gemäß Anspruch 1, wobei das Tensid in einem Bereich von 1 Gew.-% bis 30 Gew.-% der Körperpflegezusammensetzung, vorzugsweise von 5 Gew.-% bis 25 Gew.-% und besser von 7 Gew.-% bis 20 Gew.-% vorhanden ist.

6. Verwendung gemäß Anspruch 1, wobei die Körperpflegezusammensetzung ein Shampoo oder eine Körperreinigungszusammensetzung ist.

7. Ein Verfahren zum Erhöhen der Ablagerung von Silikon auf Haar, das Folgendes beinhaltet:
Dispergieren des Siikons in einer wässrigen Dispersion, die ein Ethylen-Acrylsäurecopolymer beinhaltet;
Einschließen der wässrigen Dispersion in einer Körperpflegezusammensetzung, die kein kationisches Polymer aufweist; und
Auftragen der Körperpflegezusammensetzung auf Haar.

8. Verfahren gemäß Anspruch 7, wobei die Ablagerung um mindestens 20 % erhöht wird.

## Revendications

1. Utilisation d'une dispersion aqueuse comprenant du silicone et un copolymère acide acrylique-éthylène afin d'augmenter le dépôt de silicone sur les cheveux d'une composition de soin personnel comprenant ladite dispersion aqueuse et au moins un tensio-actif acceptable d'un point de vue cosmétique ; ladite composition de soin personnel ne présentant pas de polymère cationique.

2. L'utilisation de la revendication 1, dans laquelle le silicone est présent en une proportion allant de 5 % en poids à 35 % en poids par rapport au poids de la dispersion aqueuse, préférablement en une proportion allant de 10 % en poids à 25 % en poids.

3. L'utilisation de la revendication 1, dans laquelle le copolymère acide acrylique-éthylène est présent en une proportion allant de 0,5 % en poids à 15 % en poids par rapport au poids de la dispersion aqueuse, préférablement en une proportion allant de 2 % en poids à 10 % en poids.

4. L'utilisation de la revendication 1, dans laquelle la dispersion aqueuse est présente en une proportion allant de 0,1 % en poids à 10 % en poids de matières solides par rapport au poids de la composition de soin personnel, préférablement en une proportion allant de 0,5 % en poids à 5 % en poids.

5. L'utilisation de la revendication 1, dans laquelle le tensio-actif est présent en une proportion allant de 1 % en poids à 30 % en poids par rapport au poids de la composition de soin personnel, préférablement de 5 % en poids à 25 % en poids, et plus préférablement de 7 % en poids à 20 % en poids.

6. L'utilisation de la revendication 1, dans laquelle la composition de soin personnel est un shampoing ou une composition de nettoyant corporel.

7. Une méthode d'augmentation du dépôt de silicone sur les cheveux, comprenant :
la dispersion du silicone dans une dispersion aqueuse comprenant un copolymère acide acrylique-éthylène ;
l'inclusion de la dispersion aqueuse dans une composition de soin personnel qui ne présente pas de polymère cationique ; et
l'application de la composition de soin personnel sur les cheveux.

8. La méthode de la revendication 7, dans laquelle le dépôt est augmenté d'au moins 20 %.
